# FASCICULE DE BREVET EUROPEEN

(11) **EP 2 956 878 B1**
(45) Date de publication et mention de la délivrance du brevet: **06.10.2021**
(21) Numéro de dépôt: 14710022.6
(22) Date de dépôt: 17.02.2014
(51) Int. Cl.: G16H 40/40, G16H 40/63, G16H 40/67

(54) **PROCEDE ET SYSTEME DE SURVEILLANCE A DISTANCE D'UN DISPOSITIF MEDICAL LOGICIEL**
VERFAHREN UND SYSTEM ZUR FERNÜBERWACHUNG EINER MEDIZINISCHEN SOFTWAREVORRICHTUNG
METHOD AND SYSTEM FOR REMOTELY MONITORING A MEDICAL SOFTWARE DEVICE

(30) Priorité: 15.02.2013 FR 1351333
(43) Date de publication de la demande: 23.12.2015
(73) Titulaire: Voluntis, 75017 Paris (FR)
(72) Inventeur: VIAL, Etienne, 78300 Poissy (FR); MARMOT, Romain, Belmont, MA 02478 (US)
(74) Mandataire: Plasseraud IP
(86) Numéro de dépôt international: PCT/FR2014/050321
(87) Numéro de publication internationale: WO 2014/125235

(56) Documents cités:
- WO-A2-2009/137699
- US-A1- 2011 320 595
- US-A1- 2011 320 595
- US-B1- 7 389 144
- VAISHALI HEGDE ET AL: "Case study - Post market product monitoring system", RELIABILITY AND MAINTAINABILITY SYMPOSIUM (RAMS), 2011 PROCEEDINGS - ANNUAL, IEEE, 24 January 2011 (2011-01-24), pages 1-5, XP031943249, DOI: 10.1109/RAMS.2011.5754520 ISBN: 978-1-4244-8857-5
- Anonymous: "Remote administration - Wikipedia", , 18 January 2013 (2013-01-18), XP055801712, Retrieved from the Internet: URL:https://en.wikipedia.org/w/index.php?t itle=Remote_administration&oldid=533679866 [retrieved on 2021-05-05]

## Description

### Domaine technique et Art antérieur

L'objet de la présente invention a trait au domaine des dispositifs médicaux, et porte plus particulièrement sur les dispositifs médicaux dits logiciels.

Un logiciel peut désormais être considéré comme un dispositif médical, ceci pour autant qu'il soit destiné par le fabricant à être utilisé spécifiquement à des fins notamment diagnostique et/ou thérapeutique.

Le logiciel en tant que dispositif médical peut donc influer directement sur la santé du patient. En conséquence, un tel logiciel est susceptible de représenter un risque pour le patient si l'une de ses fonctionnalités est défaillante.

Prenons l'exemple simple d'un patient asthmatique : l'utilisation d'un dispositif médical logiciel destiné à accompagner le patient dans le traitement de l'asthme peut permettre la détermination de la dose recommandée d'un bronchodilatateur pour éviter ou limiter la crise d'asthme ; ceci peut se faire par exemple en fonction notamment :
- d'une information relative au Débit Expiratoire de Pointe (DEP) mesuré à l'aide d'un appareil de mesure adapté, et
- d'une prescription médicale établie par un médecin spécialiste prenant en considération des informations relatives au patient (par exemple : type d'asthme, âge, poids, activité régulière du patient, antécédent, etc.).

On comprend ici que ce dosage du bronchodilatateur doit être conforme à la prescription médicale et à l'état de santé du patient ; une erreur dans la détermination de ce dosage due par exemple à un « *bug* » informatique, une incompatibilité de la version du logiciel avec son environnement informatique ou encore une erreur dans le DEP mesuré, peut avoir des conséquences sur la santé du patient, une telle erreur de dosage pouvant dans certains cas extrêmes conduire au décès du patient.

Bien évidemment, le traitement de l'asthme est un exemple parmi d'autres exemples : il est en effet possible de prévoir un dispositif médical logiciel pour accompagner le patient dans le traitement d'autres types de pathologies telles que par exemple le traitement de troubles cardiovasculaires, de la sclérose en plaques (SEP), ou encore de l'épilepsie.

Pour limiter les risques liés à l'utilisation d'un dispositif médical logiciel, il est impératif que ce dernier soit utilisé dans les conditions et aux fins prévues par le fabricant ; ceci permet de pas compromettre l'état clinique ni la sécurité du patient.

Cependant, malgré les différentes réglementations nationales, communautaires et internationales, et les procédures qualité mises en place chez les fabricants pour limiter les risques, il est très difficile, voire impossible, de reproduire avant la mise sur le marché l'ensemble des situations auxquelles le dispositif médical peut être confronté.

Il existe donc des procédures pour surveiller les dispositifs médicaux après leur mise sur le marché pour limiter ces risques, ou à tout le moins prendre les mesures nécessaires lorsqu'un risque potentiel ou un incident a été identifié.

On parle de « *matériovigilance* »*.*

Cette « *matériovigilance* » est généralement orchestrée par les autorités de santé pour recueillir et analyser les incidents et les risques, qui se sont produits ou risqueraient de se produire, avec des dispositifs médicaux déjà mis sur le marché.

Dans le cas spécifique du dispositif médical logiciel, cette « *matériovigilance* » implique de surveiller l'ensemble des risques liés à la défaillance ou le mésusage de l'une des fonctionnalités proposées par le logiciel.

Les solutions informatiques sont en effet, par nature, sujettes à des anomalies et des « *bugs* » informatiques, souvent difficilement détectables.

Ceci implique l'identification systématique de tout problème informatique ou matériel lié au dispositif médical logiciel : une incompatibilité informatique ou logicielle, une mise à jour erronée, un système d'exploitation obsolète, une horloge désynchronisée, un problème d'interopérabilité, un modèle d'appareil auxiliaire (par exemple un appareil de mesure ou une sonde) non supporté, etc.

Une fois que le problème est identifié, celui-ci est étudié suivant des procédures de qualité normalisées et encadrées par les autorités compétentes. Au cours de ces études sont évalués les risques encourus par le patient.

A l'issue de ces études, les autorités compétentes peuvent exiger ou non le déclenchement d'une procédure d'action corrective (connue sous le terme anglais « *field safety corrective action* ») ou de rappel (connue sous le terme anglais « *medical device recall* »)*.*

Le document US 2009/0282244 tente d'apporter une solution à cette problématique de « *matériovigilance* », et propose avec chaque dispositif médical la distribution d'une clé d'authentification.

La technologie déployée dans ce document nécessite l'envoie à un serveur informatique de cette clé avant chaque utilisation. De son côté, le serveur gère une liste préétablie de clés pour lesquelles l'accès est autorisé. Une fois l'autorisation délivrée par le serveur après vérification de la clé sur la liste, le patient peut utiliser librement son dispositif médical.

La solution proposée ici est particulièrement lourde à gérer sur le plan informatique, ceci tant au niveau du serveur qu'au niveau de l'utilisateur lui-même.

Par ailleurs, une telle solution nécessite un accès permanent au réseau, ce qui est contraignant notamment pour un patient qui voyage à l'étranger ou qui est dans une zone non couverte par le réseau.

La demanderesse soumet que l'authentification par clé proposée dans ce document permet l'utilisation effective du dispositif médical en activant l'ensemble des fonctionnalités logicielles dudit dispositif médical. Cependant, avec la technologie proposée dans ce document, lorsque l'authentification échoue, le dispositif médical dans son ensemble est désactivé ; une telle approche n'est pas acceptable dans les situations pour lesquelles certaines des fonctionnalités logicielles seraient vitales pour le patient.

Le document WO 2009/137699 propose une solution sensiblement identique à celle proposée dans le document US 2009/0282244. Plus particulièrement, dans ce document, des informations dites de sécurité associées à un kit sont enregistrées sur un support et permettent à un contrôleur d'authentifier la provenance du kit de traitement médical.

Dans ce document, l'authentification vise simplement à vérifier que le kit destiné à être utilisé ici est compatible avec le dispositif médical. Si l'authentification est positive, le contrôleur autorise la délivrance du traitement du kit par le dispositif médical.

Cette solution permet de vérifier l'appairage du kit avec le dispositif médical, les informations de sécurité jouant ici le rôle d'une clé d'authentification.

L'approche proposée dans ce document est donc intéressante lorsque l'environnement informatique du dispositif médical n'évolue pas, ce qui est le cas généralement des dispositifs médicaux classiques. En effet, l'environnement informatique et logiciel d'un dispositif médical classique n'évolue pas, car cet environnement fait partie intégrante du dispositif.

De façon contraire, l'environnement informatique et logiciel d'un dispositif médical logiciel est non stable et non maîtrisé. Un dispositif médical logiciel au sens de la présente invention peut en effet subir de nombreuses évolutions informatiques et logicielles telles que par exemple des mises à jour, une nouvelle version logicielle, etc.

Ceci est d'autant plus vrai lorsque les dispositifs médicaux logiciels sont distribués et intégrés sur des terminaux destinés au grand public tels que des « *smartphones* » ou des tablettes, de tels terminaux n'étant pas dédiés initialement à un usage médical.

La demanderesse soumet que les solutions proposées dans le document WO 2009/137699 ainsi que dans le document US 2009/0282244 ne peuvent pas prendre pas en considération les évolutions de l'environnement informatique et matériel d'un dispositif médical logiciel.

Comme expliqué précédemment, cette prise en considération en continu des évolutions de l'environnement informatique et matériel d'un dispositif médical est une contrainte nouvelle qui apparaît avec l'émergence des dispositifs médicaux logiciels ; une telle contrainte ne peut être ignorée dans la mesure où la santé du patient peut dépendre de ces évolutions ; en effet, comme expliqué ci-dessus, l'ensemble de ces évolutions peut avoir une influence négative sur le bon fonctionnement de l'une ou l'autre des fonctionnalités proposées par le logiciel.

En tout état de cause, aucun de ces deux documents ne permet de contrôler en continu les capacités de fonctionnement d'une fonctionnalité logicielle d'un dispositif médical logiciel, et de sonder les éventuelles évolutions non maitrisées des données extrinsèques dudit dispositif.

La demanderesse soumet ainsi que le document WO 2009/137699 ne permet pas l'identification systématique d'un problème informatique ou matériel lié au dispositif médical logiciel tel que par exemple une incompatibilité informatique ou logicielle, une mise à jour erronée, un système d'exploitation obsolète, une horloge désynchronisée, un problème d'interopérabilité, un modèle d'appareil auxiliaire non supporté, etc.

En effet, comme indiqué ci-dessus, l'approche proposée dans ce document WO 2009/137699 ne permet que de vérifier que le kit destiné à être utilisé ici est compatible avec le dispositif médical.

Des exemples et des modes de réalisation de l'art antérieur peuvent être trouvés dans le brevet US 7,389, 144 B1. Ce document ne divulgue cependant pas une étape d'émission d'un message de validation, lorsqu'aucun risque n'est identifié, comportant un ordre pour maintenir actives certaines fonctionnalités logicielles pendant une période de validité prédéterminée, dans lequel au moins une de ces fonctionnalités logicielles est désactivée à la fin de la période de validité prédéterminée si aucun autre message de validation n'a été reçu par le dispositif médical.

Il n'existe donc pas à ce jour de solution technique efficace et performante pour surveiller efficacement les dispositifs médicaux logiciels.

Le récent statut du logiciel en tant dispositif médical à part entière requiert donc de nouvelles contraintes auxquelles doit fait face l'ensemble des acteurs de la santé.

### Résumé et objet de la présente invention

La présente invention vise à améliorer la situation actuelle en proposant un procédé de surveillance d'un dispositif médical, un système de surveillance d'un dispositif médical, un serveur informatique pour la surveillance d'un dispositif médical et un dispositif médical à disposition d'un patient tels que décrits dans les revendications indépendantes. Des modes de réalisation sont décrits dans les revendications dépendantes.

Un des objectifs de la présente invention est de permettre la surveillance d'un dispositif médical logiciel pour éviter notamment que ne se produisent (ou reproduisent) des incidents ou des risques d'incidents mettant en cause une ou plusieurs fonctionnalités du logiciel.

La présente invention vise également à permettre une gestion optimale des procédures de rappel ou d'action corrective des dispositifs médicaux logiciels.

A cet effet, l'objet de la présente invention porte sur un procédé de surveillance qui est mis en œuvre par des moyens informatiques, et qui permet la surveillance d'un ou plusieurs dispositifs médicaux à disposition respectivement d'un ou plusieurs patients.

Selon la présente invention, le dispositif médical comporte un terminal de communication qui met en œuvre des fonctionnalités logicielles pour accompagner le patient dans un traitement thérapeutique prédéterminé.

Il s'agit de fonctionnalités permettant par exemple : le dosage d'un bronchodilatateur pour le traitement de l'asthme, le suivi de l'observance du traitement contre certaines maladies auto-immunes, le dosage d'un anticoagulant pour le traitement de certains troubles cardiaques, le dosage en carbamazépin pour le traitement de l'épilepsie, etc.

Bien évidemment, ces exemples de fonctionnalités ne présentent en aucun cas un caractère limitatif ; ces exemples sont purement illustratifs et l'homme du métier comprend ici que d'autres fonctionnalités relevant par exemple du domaine du dispositif médical peuvent être envisagées dans le cadre de la présente invention pour accompagner un patient dans le traitement d'autres pathologies.

Selon la présente invention, le procédé de surveillance comporte une étape de réception de données courantes d'exécution des fonctionnalités logicielles, ces données courantes provenant du dispositif médical.

De préférence, ces données courantes sont transmises de façon proactive par le dispositif médical, lorsque par exemple le patient fait fonctionner le dispositif médical.

Les données courantes correspondent ici à des données contenant au moins une information relative à l'état notamment :
du terminal de communication lui-même (par exemple le numéro de modèle du terminal, son système d'exploitation « *operating system* », son horloge interne, etc.), et/ou
- de l'application logicielle qui supporte les différentes fonctionnalités logicielles (par exemple la version de l'application logicielle, ses dernières mises à jour, etc.), et/ou
- du patient (son âge, son genre, son activité, son pays, la pathologie à traiter, etc.).

Ces données courantes sont destinées à être exécutées et/ou utilisées par les fonctionnalités logicielles du dispositif médical.

La collecte de ces données courantes est caractéristique de la présente invention pour le traitement qui suit.

En effet, le procédé de surveillance selon la présente invention comporte une étape de test qui consiste à comparer ces données courantes à des données de référence. Cette comparaison se fait selon au moins une règle de vigilance prédéterminée qui est associée à un risque pour le patient. On comprend ici que le risque est lié à l'exécution d'au moins l'une des fonctionnalités logicielles avec les données courantes. Avantageusement, chaque règle de vigilance est liée à un risque identifié dans l'exécution d'au moins l'une des fonctionnalités du dispositif médical avec les données courantes. Cette règle de vigilance a été établie en amont du procédé lorsque par exemple un risque a été identifié ou un incident a été reporté.

Ce test permet ainsi d'identifier un risque lorsque les données courantes ne satisfont pas les conditions définies par la règle de vigilance : un risque est identifié lorsque par exemple le résultat de la comparaison entre les données courantes et les données de référence aboutit à une valeur attendue ou une valeur qui est supérieure à un seuil de tolérance prédéterminé.

La demanderesse soumet ici qu'aucun document de l'état de la technique ne prévoit un test de comparaison basé sur au moins une règle de vigilance.

Bien évidemment, il faut comprendre ici que l'homme du métier dans la mise en œuvre de cette étape aura le choix d'implémenter plusieurs types de test pour comparer les données courantes à des données de référence et identifier un risque en fonction de cette comparaison et d'une règle de vigilance prédéterminée.

Cette comparaison peut par exemple correspondre sur le plan mathématique au calcul d'une différence, d'une valeur absolue d'une différence, d'un ratio, etc.

Le résultat de cette comparaison, qui aboutit grâce à la règle de vigilance à l'identification ou non d'un risque, permet de mener une action adaptée : à cet effet, le procédé de surveillance selon la présente invention comporte une étape d'émission au cours de laquelle un message de vigilance est émis lorsqu'un risque est identifié ; ceci est particulièrement avantageux en comparaison aux solutions de l'état de la technique (notamment dans le document WO 2009/137699) dans lesquelles le dispositif médical est systématiquement désactivé si la clé d'authentification aboutit à un test de comparaison négatif.

Ainsi, grâce à cette succession d'étapes techniques, caractéristique de la présente invention, le procédé de surveillance permet de collecter des informations relatives à l'état intrinsèque et/ou extrinsèque du dispositif médical (état du terminal de communication, de l'application logicielle, et/ou du patient), et de comparer ces informations à des données de référence attendues. Cette comparaison permet en fonction d'au moins une règle de vigilance prédéterminée d'identifier la présence ou non d'un risque potentiel, un tel risque étant lié à au moins l'une des fonctionnalités du dispositif médical logiciel.

La demanderesse observe qu'est déterminée au moins une règle de vigilance pour chaque risque potentiel.

En d'autres termes, le test effectué selon la présente invention permet d'identifier un risque potentiel pour le patient dans l'exécution d'une ou plusieurs fonctionnalités du dispositif médical logiciel avec les données courantes, ce risque étant dû par exemple à une incompatibilité informatique entre la version logicielle et l'environnement informatique proposé par le terminal de communication.

De préférence, lorsqu'un tel risque est identifié, le message de vigilance est émis à destination du dispositif médical notamment pour avertir le patient.

Ce message de vigilance peut également être émis à destination du médecin traitant et/ou de la personne en charge du suivi médical du patient et/ou de l'observance du traitement (par exemple une infirmière).

Le message de vigilance comporte un ordre pour désactiver à distance la ou les fonctionnalités logicielles lorsqu'un tel risque est identifié.

Ceci permet donc de désactiver à distance la ou les fonctionnalités qui sont à l'origine du risque, ce risque étant directement lié à l'exécution de cette ou ces fonctionnalités avec les données courantes.

Ce mode de réalisation est particulièrement adapté pour cibler les dispositifs médicaux qui présentent un risque pour le patient en raison par exemple d'une défaillance logicielle. Un tel mode permet d'éviter les campagnes de rappel qui sont coûteuses, longues et fastidieuses à mettre en place, et qui sont lancés habituellement sur tous les dispositifs, lorsque ce genre de risque est identifié.

Cette désactivation d'au moins une fonctionnalité logicielle peut se faire jusqu'à ce qu'un correctif soit apporté à ladite fonctionnalité : une mise à jour informatique par exemple.

Cette désactivation partielle de la ou des fonctionnalités concernés par le risque potentiel permet une véritable granularité qu'on ne retrouve pas dans l'état de la technique.

Lorsqu'aucun risque n'est identifié, un message de validation est émis ; ce message de validation comporte un ordre pour maintenir actives les fonctionnalités logicielles. Un tel message de validation permet de garantir que les fonctionnalités actives ne peuvent pas entraîner un quelconque risque (déjà connu et répertorié). Ceci renforce la sécurité du patient dans l'utilisation du dispositif médical.

De préférence, les fonctionnalités logicielles du dispositif médical sont par défaut désactivées (lors d'une première utilisation suite à une mise à jour par exemple). Dans cette variante, le message de validation permet donc l'activation de ces fonctionnalités pour leur première utilisation.

Le message de validation comporte un ordre pour maintenir actives les fonctionnalités logicielles pendant une période de validité prédéterminée.

Dans cette variante, il est envisageable de prévoir que le dispositif médical comporte des moyens informatiques spécifiques permettant de désactiver automatiquement la ou les fonctionnalités correspondantes, lorsque cette période de validité est dépassée. Ce mécanisme de vigilance, interne au dispositif médical, permet de limiter les risques, par exemple lorsque le patient n'est plus connecté au réseau.

Ce mécanisme de vigilance utilise par exemple une horloge interne.

Dans un mode de réalisation avantageux, la règle de vigilance comporte un critère de compatibilité des fonctionnalités logicielles avec un environnement logiciel et/ou matériel.

Ce mode de réalisation permet de vérifier que les fonctionnalités logicielles qui sont implémentées sur le dispositif médical fonctionnent correctement avec l'environnement logiciel et/ou matériel du dispositif médical. Une incompatibilité entre ces fonctionnalités et l'environnement logiciel et/ou matériel du dispositif médical pourrait en effet être à l'origine d'un « *bug* » informatique ou d'une défaillance dans l'exécution d'au moins une des fonctionnalités logicielles : par exemple, une erreur dans le calcul du dosage du bronchodilatateur.

Ce mode de réalisation permet donc d'identifier les risques potentiels liés à une telle incompatibilité, et le cas échéant de désactiver la ou les fonctionnalités correspondantes si le risque potentiel est jugé élevé par les autorités compétentes.

Dans un autre mode de réalisation avantageux qui peut éventuellement être combiné avec le précédent mode, les données courantes contiennent au moins une information relative à une version des fonctionnalités logicielles.

Les fonctionnalités logicielles peuvent en effet subir des mises à jour régulières. Dans ce cas, il est possible qu'une mise à jour logicielle ne soit pas compatible avec l'environnement logiciel et/ou matériel du dispositif médical : par exemple, l'environnement est obsolète et ne supporte la nouvelle version du logiciel. Dans ce mode, l'information relative à une version des fonctionnalités logicielles est donc vérifiée pour identifier un risque potentiel, et le cas échéant il est indiqué au patient dans le message de validation qu'une mise à jour est nécessaire pour utiliser correctement le dispositif médical.

Dans un autre mode de réalisation avantageux qui peut éventuellement être combiné avec l'un et/ou l'autre des deux précédents modes, les données courantes comportent une information de version d'environnement informatique d'exécution des fonctionnalités logicielles.

Dans ce cas, cette information est comparée avec l'information relative à l'environnement informatique du dispositif médical.

Dans un autre mode de réalisation avantageux qui peut éventuellement être combiné avec l'un et/ou l'autre des trois précédents modes, les données courantes comportent une information de modèle de terminal de communication.

Dans ce cas, on comprend que le test peut consister par exemple à comparer cette information avec l'information relative aux modèles de terminal de communication qui sont considérés comme pouvant supporter les fonctionnalités logicielles. Si le modèle de terminal de communication du dispositif médical ne fait pas partie de cette liste (c'est ici la règle de vigilance qui est établie), alors un risque potentiel est identifié : ce risque identifié ici est lié directement à l'utilisation de ce modèle de terminal.

De préférence, le dispositif médical comporte, outre le terminal de communication, un dispositif auxiliaire de mesure médicale tel que par exemple une sonde permettant de mesure le DEP du patient ou encore une sonde cardiaque permettant de mesurer la fréquence cardiaque du patient, ou sa tension.

Dans cette variante, les données courantes comportent une information de modèle de ce dispositif auxiliaire. On comprend donc ici que la règle de vigilance comporte un critère de compatibilité entre le dispositif auxiliaire et le dispositif médical. La comparaison établie lors de l'étape de test consiste dans ce cas à comparer cette information avec les données de références relatives aux différents modèles attendus, en prenant en considération ce critère de compatibilité. Si le modèle du dispositif auxiliaire n'est pas compatible avec le dispositif médical, ceci peut signifier qu'il existe un risque que les mesures réalisées par le dispositif auxiliaire ne soient pas fiables ; dans un cas, une désactivation de la fonctionnalité « *mesure avec le dispositif auxiliaire de mesure* » est requise.

De préférence, le dispositif médical comporte :
- au moins un capteur d'activité du patient, et
- un module d'interprétation de cette activité pour déterminer une donnée d'activité du patient.

Dans cette variante, les données courantes comportent cette donnée d'activité du patient.

De la même façon, le dispositif médical peut également comporter au moins un capteur, dit biologique, permettant de mesurer un signal biologique relatif au patient, un tel signal comprenant une information biologique relative à l'état biologique du patient. Dans cette variante, le module d'interprétation est configuré pour déterminer une donnée biologique du patient.

Dans cette variante, les données courantes comportent cette donnée biologique du patient.

Corrélativement, l'objet de la présente invention porte sur un programme d'ordinateur qui comporte des instructions adaptées pour l'exécution des étapes du procédé de surveillance tel que décrit ci-dessus, ceci notamment lorsque ledit programme d'ordinateur est exécuté par au moins un processeur.

Un tel programme d'ordinateur peut utiliser n'importe quel langage de programmation, et être sous la forme d'un code source, d'un code objet, ou d'un code intermédiaire entre un code source et un code objet, tel que dans une forme partiellement compilée, ou dans n'importe quelle autre forme souhaitable.

De même, l'objet de la présente invention porte sur un support d'enregistrement lisible par un ordinateur sur lequel est enregistré un programme d'ordinateur comprenant des instructions pour l'exécution des étapes du procédé de surveillance tel que décrit ci-dessus.

D'une part, le support d'enregistrement peut être n'importe quel entité ou dispositif capable de stocker le programme. Par exemple, le support peut comporter un moyen de stockage, tel qu'une mémoire ROM, par exemple un CD-ROM ou une mémoire ROM de type circuit microélectronique, ou encore un moyen d'enregistrement magnétique, par exemple une disquette de type « *floppy disc* » ou un disque dur.

D'autre part, ce support d'enregistrement peut également être un support transmissible tel qu'un signal électrique ou optique, un tel signal pouvant être acheminé via un câble électrique ou optique, par radio classique ou hertzienne ou par faisceau laser autodirigé ou par d'autres moyens. Le programme d'ordinateur selon l'invention peut être en particulier téléchargé sur un réseau de type Internet.

Alternativement, le support d'enregistrement peut être un circuit intégré dans lequel le programme d'ordinateur est incorporé, le circuit intégré étant adapté pour exécuter ou pour être utilisé dans l'exécution du procédé en question.

L'objet de la présente invention porte également sur un système de surveillance d'un dispositif médical à disposition d'un patient, ledit dispositif médical comportant un terminal de communication mettant en œuvre des fonctionnalités logicielles pour accompagner le patient dans un traitement thérapeutique prédéterminé.

Selon la présente invention, le système de surveillance comporte :
- un récepteur qui est configuré pour recevoir du dispositif médical des données courantes d'exécution des fonctionnalités logicielles,
- un circuit de test qui est configuré pour comparer les données courantes à des données de référence selon au moins une règle de vigilance prédéterminée associée à un risque pour le patient, ce risque étant lié à l'exécution d'au moins l'une des fonctionnalités logicielles avec les données courantes, et
- un émetteur qui est configuré pour émettre un message de vigilance lorsqu'un tel risque est identifié.

Avantageusement, le système de surveillance comporte des moyens informatiques qui sont configurés pour permettre la mise en œuvre des différentes étapes du procédé de surveillance tel que décrit ci-dessus.

L'objet de la présente invention porte également sur un serveur informatique pour la surveillance d'un dispositif médical mis à disposition d'un patient, ledit serveur informatique comportant un système de surveillance tel que décrit ci-dessus.

L'objet de la présente invention porte aussi sur un dispositif médical à disposition d'un patient ; ce dispositif médical est configuré pour permettre la mise en œuvre du procédé de surveillance par le système de surveillance décrit ci-dessus.

Selon la présente invention, le dispositif médical comporte :
- un terminal de communication qui met en œuvre des fonctionnalités logicielles pour accompagner le patient dans un traitement thérapeutique prédéterminé,
- un moyen de transmission qui est configuré pour transmettre des données courantes d'exécution desdites fonctionnalités logicielles à destination d'un système de surveillance tel que décrit ci-dessus, et
- un moyen de vigilance qui est configuré pour désactiver au moins une fonctionnalité logicielle à la réception d'un message de vigilance émis lorsqu'un risque a été identifié par le système de surveillance, ledit message de vigilance comportant un ordre pour désactiver ladite au moins une fonctionnalité logicielle.

Avantageusement, le moyen de transmission est configuré pour transmettre les données courantes de façon périodique (c'est-à-dire selon une périodicité déterminée, par exemple toutes les heures, tous les jours, toutes les semaines, etc.)

Le système de surveillance est configuré pour émettre un message de validation lorsqu'aucun risque n'a été identifié. Dans ce cas, le moyen de vigilance est configuré pour désactiver au moins une des fonctionnalités logicielles du dispositif médical lorsqu'aucun message de validation provenant du système de surveillance n'a été reçu.

Le message de validation comporte un ordre pour maintenir actives la ou les fonctionnalités logicielles pendant une période de validité prédéterminée. Lorsqu'aucun message de validation provenant du système de surveillance n'a été reçu pendant une période supérieure à la période de validité, alors le moyen de vigilance est configuré pour désactiver au moins une des fonctionnalités logicielles.

Le dispositif médical peut également comporter une horloge interne pour désactiver la ou les fonctionnalités lorsque la période de validité est expirée ou lorsqu'aucun message de validation n'a été reçu pendant une période prédéterminée.

Ainsi, l'objet de la présente invention, par ses différents aspects fonctionnels et structurels décrits ci-dessus, permet la surveillance à distance d'au moins un dispositif médical logiciel pour notamment :
- avertir le patient d'un risque potentiel lié à l'utilisation du dispositif médical logiciel et notamment un risque lié l'exécution d'au moins l'une des fonctionnalités logicielles de ce dispositif médical logiciel, et
- gérer de manière optimale les actions correctives et/ou les actions de rappel liées auxdits dispositifs lorsqu'un tel risque est identifié.

### Brève description des figures annexées

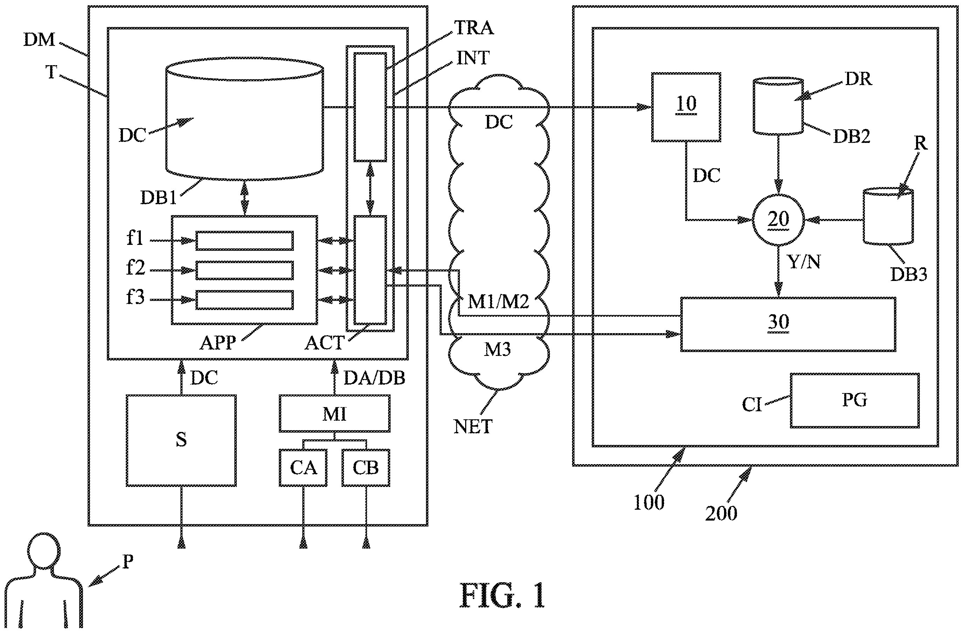

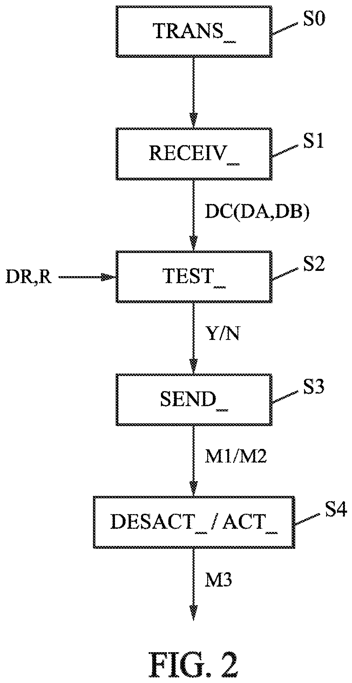

D'autres caractéristiques et avantages de la présente invention ressortiront de la description ci-dessous, en référence aux figures 1 et 2 annexées qui en illustrent un exemple de réalisation dépourvu de tout caractère limitatif et sur lesquelles :
- la figure 1 représente de façon schématique un serveur informatique comprenant un système de surveillance à distance d'au moins un dispositif médical logiciel conforme à un exemple de réalisation de la présente invention ; et
- la figure 2 représente un organigramme illustrant le procédé de surveillance conforme à un exemple de réalisation de la présente invention.

### Description détaillée de différents modes de réalisation avantageux

Un procédé de surveillance d'un dispositif médical logiciel DM ainsi que le système informatique de surveillance 100 qui lui est associé vont maintenant être décrits dans ce qui suit en faisant référence conjointement aux figures 1 et 2.

Permettre la surveillance d'un ou plusieurs dispositifs médicaux logiciels DM dans une problématique de « *matériovigilance* » est un des objectifs de la présente invention.

Dans l'exemple décrit ici, le dispositif médical logiciel DM dont il est question est configuré pour permettre l'accompagnement d'un patient P souffrant d'hypertension artérielle.

Dans cet exemple, pour traiter cette pathologie, le patient P doit prendre un médicament du type Inhibiteur de l'Enzyme de Conversion, noté ci-après IEC. Ce médicament nécessite une adaptation régulière de la posologie qui est fonction notamment de la tension artérielle systolique du patient P.

Bien évidemment, comme expliqué précédemment, cet exemple est purement illustratif et ne présente en aucun cas un caractère limitatif : l'homme du métier comprend ici que le patient P peut être accompagné dans le traitement de tout autre type de pathologie en utilisant un dispositif médical DM au sens de la présente invention :
l'exemple décrit ici peut ainsi être transposé sans aucune difficulté au traitement d'une autre pathologie telle que par exemple l'asthme, la SEP, etc.

Dans l'exemple décrit ici et comme illustré en figure 1, le dispositif médical logiciel DM comporte donc un terminal de communication T tel que par exemple un « *smartphone* » ou une tablette numérique sur lequel est intégrée une application logicielle APP supportant plusieurs fonctionnalités f1, f2 et f3 permettant l'accompagnement du patient P dans son traitement.

Dans l'exemple décrit ici, on relève, parmi les fonctionnalités f1, f2 et f3, la fonctionnalité f1 qui porte sur un algorithme relatif au calcul du dosage de l'IEC. Cet algorithme calcule le dosage d'IEC en prenant en considération notamment les variations de l'hypertension du patient P.

Dans l'exemple décrit ici et comme illustré en figure 1, le dispositif médical DM comporte un dispositif auxiliaire tel qu'une sonde cardiaque S spécialement configurée pour mesurer la tension artérielle systolique du patient P et identifier les variations de l'hypertension. Cette sonde S consiste par exemple en un appareil d'auto-mesure de la tension.

On considère que dans l'exemple décrit ici cette sonde S porte le numéro de modèle « X-1 ».

Dans l'exemple décrit ici, chaque jour avant de prendre sa dose de traitement, le patient P mesure sa tension à l'aide de cette sonde S pour connaître la dose d'IEC qu'il doit prendre.

Dans le cas d'un patient souffrant d'hypertension, il est généralement attendu que cette mesure soit comprise entre 10 et 20 centimètres de mercure (cm Hg).

Dans un premier exemple de réalisation décrit ici et pour illustrer un des nombreux avantages de la présente invention, le patient P doit changer les piles de la sonde S qui sont usées.

Or, ce changement de piles provoque ici une remise aux paramètres d'usine de la sonde S ; dans l'hypothèse de travail développé ici qui est purement illustrative, ce problème matériel (remise aux paramètres d'usine) n'avait pas été identifié par le fabricant avant la mise sur le marché.

Ainsi, dans cet exemple, suite à cette remise aux paramètres d'usine, l'unité de mesure devient le millimètre de mercure (mm Hg) : les valeurs de la mesure de tension sont désormais comprises entre 100 à 200 (mm Hg) au lieu des 10 à 20 (cm Hg) attendues.

Suite à ce changement de piles, le patient P réalise sa mesure et reporte dans l'application APP la valeur qu'il lit sur la sonde S, à savoir par exemple la valeur 140 (ce report de la valeur mesurée peut se faire automatiquement).

Dans l'exemple décrit ici, compte tenu de cette valeur (qui est dix fois plus élevée que la valeur attendue), l'algorithme relatif à la fonctionnalité de dosage f1 recommande au patient P une dose d'IEC qui est nettement supérieure à la dose requise : ce changement de piles de la sonde S est donc ici à l'origine d'une erreur de dosage manifeste.

A la suite de cette prise, le patient P fait un malaise et est hospitalisé.

Dans ce cas, un incident de « *matériovigilance* » est reporté immédiatement aux instances sanitaires compétentes par exemple par l'intermédiaire du médecin traitant qui remplit un formulaire adapté selon une procédure normalisée classique.

Dans l'exemple décrit ici, l'étude de l'incident révèle alors qu'il existe une faille dans le mécanisme de contrôle du champ de saisie de la tension artérielle dont la borne supérieure a été mal définie.

Pour remédier à ce problème de paramétrage informatique, il est nécessaire de procéder à une correction de l'algorithme f1 (par une mise à jour logicielle) pour modifier cette borne supérieure et ajouter une limite à ne pas dépasser pour la dose recommandée par le dispositif médical DM.

Dans cet exemple, le temps nécessaire pour la spécification, le développement, la vérification et la validation de la mise à jour de cette fonctionnalité f1 est estimé à plusieurs semaines.

Compte-tenu de la criticité des incidents rencontrés, en concertation avec les pouvoirs publics, une action est nécessaire en urgence auprès de l'ensemble des patients P souffrant d'hypertension et utilisant les dispositifs médicaux DM fonctionnant avec le dispositif auxiliaire S correspondant au modèle « X-1 ». En effet, pour ces dispositifs DM, il faut désactiver le plus rapidement possible la fonctionnalité f1 le temps qu'un correctif soit apporté.

Comme illustré en figure 1, la présente invention prévoit à cet effet un serveur informatique 200 qui comporte un système de surveillance 100 permettant la surveillance des dispositifs médicaux DM et présentant des moyens informatiques pour mener une action de vigilance.

Dans l'exemple décrit ici, et comme illustré en figures 1 et 2, le dispositif médical DM comporte un module d'interface INT qui peut de communiquer avec le système de surveillance 100 selon la présente invention.

Comme illustré en figures 1 et 2, ce module d'interface INT présente un moyen de transmission TRA qui est configuré pour transmettre pro-activement, lors d'une étape de transmission S0 via le réseau NET, des données telles que des données courantes DC qui sont propres au dispositif DM et qui sont stockées sur chaque dispositif DM dans une première base de données DB1.

Dans l'exemple décrit ici, les données courantes DC contiennent l'information relative au numéro de modèle de la sonde S.

Ces moyens de transmission TRA sont déployés sur tous les dispositifs médicaux DM permettent de collecter les données courantes DC de chaque dispositif DM.

Le système de surveillance 100 comporte de son côté un récepteur 10 permettant de recevoir lors d'une étape de réception S1 ces données DC.

Permettre l'identification des dispositifs médicaux DM fonctionnant avec une sonde S de type « X-1 » est ici l'objectif recherché.

Dans l'exemple décrit ici, et comme illustré en figures 1 et 2, le système de surveillance 100 comporte un circuit de test 20 qui est configuré pour tester, lors d'une étape de test S2, les données courantes DC collectées pour chaque dispositif médical DM en les comparant à des données de référence DR selon une règle de vigilance R.

Dans l'exemple décrit ici, les données de référence DR sont stockées sur une deuxième base de données DB2.

Dans cet exemple, les données de référence DR peuvent correspondre aux données relatives au numéro de modèle de la sonde S ; la règle de vigilance R, qui a été définie par l'administrateur du serveur 200 (selon les recommandations des autorités sanitaires) et qui est stockées sur une troisième base de données DB3, permet donc grâce à la comparaison entre les données courantes DC et les données de référence DR d'identifier tous les dispositifs DM comportant une sonde S de type « X-1 ».

Le système 100 comporte des moyens informatiques spécialement adaptés pour configurer les règles de vigilance R qu'il faut exécuter pour identifier les dispositifs DM à risque (non représentés ici).

Dans l'exemple décrit ici, la règle de vigilance R comporte donc un critère d'incompatibilité de la sonde S de type « X-1 » avec la fonctionnalité f1. La comparaison entre les données courantes DC et les données de référence DR corrélées avec cette règle de vigilance R permet ainsi de cibler les dispositifs DM comportant cette sonde S de type « X-1 » et concernés par ce problème.

Ce test par comparaison est caractéristique de la présente invention : il permet de faire efficacement une discrimination, de type « Yes or No » (Y/N), pour identifier les dispositifs DM présentant un risque.

Dans l'exemple décrit ici, et comme illustré en figures 1 et 2, le système 100 comporte un émetteur 30 qui est configuré pour émettre, lors d'une étape d'émission S3, un message de vigilance M1 à destination de chaque dispositif médical DM identifié comportant une sonde S de type « X-1 ».

Dans l'exemple décrit ici, le message de vigilance M1 est émis (par exemple par message *« push* ») pour avertir les patients P disposant d'une sonde S de type « X-1 » des risques potentiels liés à la fonctionnalité f1.

Dans l'exemple décrit ici, le message de vigilance M1 est en outre programmé pour permettre la désactivation de cette fonction f1 relative au dosage de l'IEC.

Dans l'exemple décrit ici et comme illustré en figure 1, le module d'interface INT du dispositif médical DM comporte un moyen de vigilance ACT configuré pour recevoir le message de vigilance M1 et mener les actions nécessaires en fonction de l'ordre contenu dans ce message M1 : par exemple ici, désactiver la fonctionnalité f1, lors d'une étape de désactivation S4.

Ce module de vigilance ACT peut également émettre vers le serveur 200 un message de confirmation M3 pour indiquer au système 100 que la désactivation de la fonctionnalité f1 a été correctement exécutée.

Il est également possible de prévoir l'émission d'un message d'avertissement pour avertir le médecin traitant suivant le patient P utilisant le dispositif médical DM mettant en œuvre cette fonctionnalité f1 (non représenté ici).

Ainsi, dans cet exemple, grâce à la présente invention, une fois qu'un risque a été identifié (ici, un problème de dosage selon la fonctionnalité f1 suite à un changement de piles pour le modèle de sonde « X-1 »), il est possible d'intervenir à distance et de manière granulaire, à travers le réseau NET pour modifier le statut de la fonctionnalité f1 permettant le dosage d'IEC et de la rendre inactive, lors d'une étape de désactivation S4, pour tous les dispositifs médicaux DM intégrant le modèle de sonde S de type « X-1 ».

Pour les dispositifs DM ne comportant pas le modèle de sonde S de type « X-1 », aucun risque potentiel lié à la sonde n'est identifié. Dans ce cas, un message de validation M2 est envoyé, lors de l'étape d'émission S3, à destination de ces dispositifs. Ce message M2 peut contenir un ordre pour maintenir active la fonctionnalité f1.

Comme illustré en figure 1, cette surveillance est réalisée par l'exécution d'instructions informatiques contenues dans un programme d'ordinateur PG pour mettre en œuvre l'ensemble des étapes du procédé de surveillance décrit ci-dessus. Dans cet exemple, le programme PG est enregistré sur un support informatique CI tel qu'un microprocesseur ou un circuit intégré.

L'homme du métier comprend ici que de nombreux autres exemples de réalisation avantageux peuvent également être envisagés dans le cadre de la présente invention.

Selon un autre exemple, il est possible que l'erreur dans le dosage d'IEC réalisée par la fonctionnalité f1 trouve son origine dans une incompatibilité entre le modèle de terminal de communication T et la version de l'application logicielle APP utilisée.

Par exemple, il est possible que le terminal de communication T correspondant à un « *iPhone5* » (marque déposée) mis à jour avec un système d'exploitation 6.1 ou un téléphone mobile intégrant le système d'exploitation « *Android* » (marque déposée) dans sa version 4.2 ne soit pas compatible avec la version 1.3 de l'application APP permettant l'accompagnement du patient P souffrant d'hypertension.

Dans cet exemple, on peut imaginer que la version 1.3 de l'application logicielle APP et plus particulièrement la fonctionnalité f1 relative au dosage d'IEC soit défaillante avec ces versions de terminal de communication T : ce problème d'incompatibilité informatique a par exemple été identifié au préalable par une équipe d'informaticiens chargée de la « *matériovigilance* », suite à de nombreux tests faisant suite à un malaise reporté.

Dans ce cas, une fois l'incompatibilité identifiée, une règle de vigilance R est définie par les autorités compétentes et est enregistrée par l'administrateur sur la troisième base de données DB3.

Le système de surveillance 100 déploie ensuite le procédé décrit ci-dessus pour identifier tous les dispositifs médicaux DM comportant un « *iPhone5* » avec le système d'exploitation 6.1 ou un téléphone mobile intégrant le système d'exploitation « *Android* » dans sa version 4.2, et fonctionnant avec la version 1.3 de l'application APP.

Cette identification comme développée ci-dessus se fait par la réceptionS1 des données courantes DC provenant de tous les dispositifs médicaux DM et par comparaison S2 de ces données courantes DC avec des données de référence DR attendues, ces données courantes DC contenant ici des informations relatives aux versions logicielles de l'application APP et aux modèles des terminaux T.

Cette comparaison se fait ici en prenant en considération une règle de vigilance R comportant le critère d'incompatibilité défini ci-dessus et associé au risque reporté dans le cadre de la « *matériovigilance* ». Par défaut, il est envisageable de prévoir systématiquement une règle de vigilance R pour désactiver tous les dispositifs DM qui comportent un terminal T avec un système d'exploitation non encore testé : Ceci évite les risques potentiels liés à la mise sur le marché d'un terminal T dont le système d'exploitation ne fonctionne pas correctement avec l'application logicielle.

Dans cet exemple, une fois que les dispositifs médicaux DM concernés par le risque ont été identifiés, un message de vigilance M1 est émis, lors d'une étape d'émission S3, à chacun des dispositifs DM ; ce message M1 peut indiquer au patient P qu'une mise à jour de la version 1.4 de l'application APP doit être téléchargée dans les plus brefs délais.

Le message de vigilance M1 peut en outre prévoir une désactivation de la fonctionnalité f1 tant que cette version 1.4 n'est pas téléchargée.

De préférence, le moyen de vigilance ACT peut comporter une horloge interne qui calcule le temps écoulé suite à la réception de ce message M1. Au bout d'un temps déterminé (24 heures par exemple), le moyen de vigilance ACT peut décider proactivement de désactiver la fonctionnalité f1 qui pose problème.

Alternativement, le moyen de vigilance ACT peut émettre un message de confirmation M3 lorsque la mise à jour a été correctement installée.

Dans cet exemple, il est également possible de prévoir l'émission d'un message de validation M2 lorsqu'aucun risque n'est identifié. Ce message de validation M2 comporte par exemple un ordre pour maintenir actives les fonctionnalités logicielles f1, f2 et/ou f3 de l'application APP, et ce pendant une période de validité déterminée. Ce message de validation M2 intervient par exemple lorsque le test révèle que la version logicielle est à jour.

Dans ce cas, l'horloge interne permet de désactiver la ou les fonctionnalités logicielles f1, f2 et/ou f3 de l'application APP si la période de temps mesurée depuis la réception du dernier message de validation M2 reçu est supérieure à la période de validité.

Dans l'exemple décrit ici, il est également prévu que les fonctionnalités f1, f2, f3 sont par défaut paramétrées initialement à l'état « désactivé ». Dans ce cas, c'est donc bien le message de validation M2 qui permet d'activer chacune de ces fonctionnalités, après que le système de surveillance 100 confirme qu'aucun risque n'est identifié.

Une telle configuration par défaut permet d'éviter les problèmes informatiques dus à une mise à jour logicielle non encore testée.

Le dispositif médical DM comporte lui aussi des moyens informatiques spécialement configurés pour limiter les risques liés à au moins l'une des fonctionnalités logicielles f1, f2 et/ou f3.

Ainsi, de façon avantageuse, le moyen de transmission TRA du dispositif médical DM transmet au système de surveillance 100 les données courantes DC de la base DB1 selon une périodicité déterminée (par exemple tous les jours).

Prenons un exemple particulier dans lequel la période de validité de la fonctionnalité f1 est d'une semaine. Si aucune donnée courante DC n'est reçue par le système de surveillance 100 pendant une période supérieure à cette période de validité (en d'autres termes, aucun message de validation M2 n'est donc reçu pendant cette période), alors, dans ce cas, le moyen de vigilance ACT désactive l'une au moins des fonctionnalités f1, f2 et/ou f3, la période de validité de la fonctionnalité f1 étant expirée.

Le dispositif médical comporte ainsi, grâce à ce moyen de vigilance ACT, un mécanisme interne qui lui permet de s'auto-prémunir du moindre risque lié à l'exécution d'une des fonctionnalités f1, f2 et/ou f3.

Ce mécanisme interne permet notamment d'éviter certains risques, notamment lorsque le patient P est situé dans une zone non-couverte par le réseau NET.

De nombreux autres exemples de réalisation sont envisageables dans le cadre de la présente invention.

Ainsi, dans un autre exemple de réalisation décrit ici à titre purement illustratif, le terminal de communication T comporte un capteur d'activité CA du patient P, par exemple un podomètre ou un système de géo-localisation.

Dans cet exemple, le terminal de communication T comporte également un module d'interprétation MI qui est configuré pour déterminer une donnée d'activité DA du patient, cette donnée d'activité DA étant relative à l'activité du patient P : par exemple ici, cette donnée DA indique une marche active du patient pendant une durée de plus d'une heure.

Dans cet exemple, les données courantes DC comportent cette donnée d'activité DA.

Or, cette donnée d'activité DA du patient P peut faire varier de façon significative le calcul de la dose d'IEC.

Dans l'exemple décrit ici à titre purement illustratif, on peut imaginer une situation dans laquelle il est fortement déconseillé de prendre une dose d'IEC à la suite d'une période d'activité physique intense. Dans cet exemple, l'application APP dans sa version actuelle est défaillante et n'intègre pas ce paramètre relatif au blocage du dosage en cas d'activité physique intense.

Le système de surveillance 100 peut remédier à cette défaillance de l'application APP en attendant qu'une mise à jour logicielle soit à disposition du patient P. Dans cet exemple, les données de référence DR attendues comportent ainsi une information indiquant que le patient P ne doit pas faire plus d'une heure de marche intensive. Dans ce cas, en déployant le procédé décrit ci-dessus, le test S2 par comparaison de la donnée courante DC avec la donnée de référence DR peut permettre d'identifier les dispositifs médicaux DM concernés et de déclencher par émission d'un message de vigilance M1 une désactivation de la fonctionnalité de dosage f1.

Bien évidemment, cet exemple ne se limite pas à ce scénario bien précis. Il est par exemple possible d'envisager une situation dans laquelle la donnée d'activité DA indique que le patient P est à l'étranger avec un décalage horaire de plusieurs heures.

Une telle information peut être pertinente par exemple si le patient P doit prendre sa dose d'IEC à heure fixe. Dans ce cas, le système de surveillance 100 prend en considération cette information pour permettre ou non l'activation de la fonctionnalité f1, en fonction de l'heure « effective » pour le patient et non l'heure réelle du fuseau horaire dans lequel il se trouve.

Cet exemple avec la donnée d'activité physique DA du patient P peut également être étendu à un exemple plus général dans lequel une donnée biologique DB ou physiologique du patient P est utilisée grâce à une mesure effectuée directement sur le patient P avec un capteur CB configuré pour mesurer un signal biologique relatif au patient P.

Parmi les nombreux autres exemples, il est possible d'envisager que le dispositif médical DM est déployé sur un terminal de communication T du type « *smartphone* » et permet le traitement de l'épilepsie par carbamazépine.

Dans cet exemple, un professionnel de santé tel que le médecin traitant saisit des données relatives aux taux plasmatiques associées à une heure définie par l'horloge interne du terminal T. Or, dans cet exemple, la synchronisation de l'horloge est désactivée. Les données saisies sont donc mal horodatées et sont interprétées comme antérieures à la prise médicamenteuse. Un mauvais horodatage peut dans ce contexte médical engendrer une décision médicale erronée par le système algorithmique APP embarqué sur le dispositif médicale DM : ceci peut par exemple provoquer un mauvais dosage selon la fonctionnalité f1.

La présente invention est configurée pour permettre une surveillance de cet horodatage et de l'heure interne du terminal T (règle de vigilance). En effet, le système 100 est configuré pour comparer l'heure courante envoyée par le dispositif DM et faisant partie des données courantes DC et l'heure du serveur 200 faisant partie des données de référence DR (cette dernière étant synchronisée avec un serveur de temps fiable).

Cette comparaison lors de l'étape de test S2 aboutit à la détection d'un problème de synchronisation et permet d'identifier la présence d'un risque potentiel lié à l'exécution de la fonctionnalité f1 relative au dosage.

Le système 100 envoie dans ce cas un message de désactivation de la fonctionnalité f1 par sécurité.

On comprend ainsi au travers des différents exemples de réalisation décrits ci-dessus que le système de surveillance 100 au sens de la présente invention, qui est mis en œuvre sur un serveur informatique 200, permet de surveiller et, le cas échéant, de désactiver, à distance par l'intermédiaire d'un réseau NET et de façon sélective, la ou les fonctionnalités logicielles f1, f2 et/ou f3 de l'application logicielle APP implémentée sur le dispositif médical DM dans les différentes circonstances où un risque potentiel pour le patient P serait identifié et serait lié à au moins l'une de ces fonctionnalités f1, f2 et/ou f3.

La présente invention permet ainsi d'identifier tout problème de nature logicielle, informatique ou matérielle pouvant entraîner un dysfonctionnement dans le dispositif médical DM, et plus précisément dans l'exécution d'au moins l'une des fonctionnalités logicielles f1, f2 et/ou f3 du dispositif médical DM avec les données courantes DC.

Cette surveillance se fait en collectant, lors d'une étape de réception S1, les données courantes DC de l'ensemble des dispositifs médicaux DM déployés, ces données courantes DC étant les données d'exécution qui sont exploitées et/ou utilisées par chacune des fonctionnalités f1, f2 et/ou f3 de chaque dispositif médical DM.

Ces données courantes DC peuvent notamment comporter :
- des informations relatives au terminal de communication T qui est le terminal hôte du dispositif médical DM. Il peut s'agir par exemple d'informations relatives au fabricant, à la marque, au modèle, à la version, au système d'exploitation, à la version du système d'exploitation, à l'identification et la version des composants (exemple : logiciel de base de données, appareil photo, résolution de l'écran, etc.), à un identifiant permettant d'assurer l'envoi de messages « *push* ». Il peut également s'agir d'informations relatives à l'horloge du terminal T : heure courante, fuseau horaire ;
- des informations relatives à l'application logicielle APP supportant les différentes fonctionnalités logicielles f1, f2 et/ou f3 : numéro de version, langue d'utilisation, date d'installation, date d'activation, configuration ;
- des informations relatives au lieu d'utilisation : pays, région ;
- des informations relatives au mode de connexion réseau/internet : opérateur téléphonique, 3G, 4G, Wifi ;
- des informations relatives au patient P : identité, âge, sexe, type de maladie, etc. ;
- des informations relatives au comportement, à un paramètre biologique du patient ou à l'activité du patient P : fréquence d'usage et heure d'usage de certaines fonctionnalités, heures d'allumage et d'éteinte du terminal T, activité physique, etc. ;
- des informations relatives au professionnel de santé suivant le patient P : médecin traitant, médecin exerçant en ville/hôpital/clinique, spécialité.

Ces données courantes DC sont ainsi comparées, lors d'une étape S2, par le circuit de test 20 du système 100 à des données de référence DR stockées sur une base de données DB2.

Cette comparaison est ensuite interprétée selon au moins une règle de vigilance R stockée sur une base de données DB3. Le traitement de cette règle de vigilance R et du résultat de cette comparaison permet de déterminer ensuite si un risque potentiel pour le patient P est susceptible de survenir lors de l'exécution d'une des fonctionnalités f1, f2 et/ou f3 avec les données courantes DC.

On comprend ici que les règles de vigilance R sont à définir en fonction de chaque cas d'espèce : ces règles R peuvent par exemple comporter un critère d'incompatibilité logicielle, matérielle, et/ou informatique, etc.

Ces règles de vigilance peuvent être implémentées et stockées sur le système 100 grâce à des moyens informatiques adaptés.

Ainsi, comme développé ci-dessus, lorsqu'un risque est identifié pour une fonctionnalité déterminée (par exemple lorsqu'un dispositif médical DM fonctionne avec une version 1.3 pour l'application APP et le modèle « *iPhone5* » fonctionnant avec un système d'exploitation version 6.1 pour le terminal hôte T), alors un message de vigilance M1 est émis à destination de l'ensemble des dispositifs médicaux DM concernés, lors d'une étape d'émission S3. Ce message M1 peut comporter un simple avertissement ou le cas échéant peut comporter un ordre pour désactiver la fonctionnalité identifiée comme potentiellement à risque.

Cette surveillance à distance par un système 100 intégré dans un serveur 200 permet de cibler précisément les dispositifs médicaux DM concernés par un risque potentiel identifié. Un tel système 100 permet de rationaliser les campagnes de rappel et les émissions de correctifs, en prenant les mesures préventives nécessaires et notamment en désactivant temporairement les fonctionnalités concernées.

Un des autres aspects de la présente invention porte sur des moyens informatiques (notamment le moyen de vigilance ACT) spécifiques au dispositif médical DM qui permettent à ce dernier grâce à un mécanisme d'horloge interne de prémunir le dispositif médical DM de tout risque potentiel lié à l'utilisation d'une fonctionnalité logicielle, lorsque par exemple le dispositif médical DM est hors connexion et n'arrive pas pendant une durée déterminée à transmettre à destination du système de surveillance 100 les données courantes DC.

Il devra être observé que cette description détaillée porte sur un exemple de réalisation particulier de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

## Revendications

1. Procédé de surveillance d'un dispositif médical (DM) à disposition d'un patient (P), dans lequel ledit dispositif médical (DM) comporte un terminal de communication (T) mettant en œuvre des fonctionnalités logicielles (f1, f2, f3) pour accompagner le patient (P) dans un traitement thérapeutique prédéterminé,
ledit procédé, mis en œuvre par des moyens informatiques, comportant les étapes suivantes :
- une étape de réception (S1) de données courantes (DC) d'exécution desdites fonctionnalités logicielles (f1, f2, f3), lesdites données courantes (DC) provenant du dispositif médical (DM),
- une étape de test (S2) desdites données courantes (DC) par comparaison à des données de référence (DR) selon au moins une règle de vigilance (R) prédéterminée associée à un risque pour le patient, ledit risque étant lié à l'exécution d'au moins l'une des fonctionnalités logicielles (f1, f2, f3) avec lesdites données courantes (DC), la règle de vigilance (R) comportant un critère de compatibilité des fonctionnalités logicielles (f1, f2, f3) avec un environnement logiciel et/ou matériel, et
- une étape d'émission (S3) d'un message de vigilance (M1) lorsqu'un tel risque est identifié lors de l'étape de test (S2) ou d'un message de validation (M2) lorsqu'aucun risque n'est identifié,
dans lequel le message de vigilance comporte un ordre pour désactiver à distance l'une au moins desdites fonctionnalités logicielles (f1, f2, f3) et le message de validation comporte un ordre pour maintenir actives les fonctionnalités logicielles (f1, f2, f3) pendant une période de validité prédéterminée, dans lequel au moins une fonctionnalité logicielle (f1, f2, f3) est désactivée à la fin de la période de validité prédéterminée si aucun autre message de validation (M2) n'a été reçu par le dispositif médical (DM).

2. Procédé selon la revendication 1, dans lequel le message de vigilance (M1) est émis à destination du dispositif médical (DM).

3. Procédé selon l'une quelconque des revendications précédentes, dans lequel les données courantes (DC) comportent :
- une information relative à une version des fonctionnalités logicielles (f1, f2, f3), et/ou
- une information de version d'environnement informatique d'exécution des fonctionnalités logicielles (f1, f2, f3), et/ou
- une information de modèle de terminal de communication (T).

4. Procédé selon l'une quelconque des revendications précédentes,
dans lequel le dispositif médical (DM) comporte, outre le terminal de communication (T), un dispositif auxiliaire (S) de mesure médicale, et
dans lequel les données courantes (DC) comportent une information de modèle de dispositif auxiliaire (S).

5. Procédé selon l'une quelconque des revendications précédentes, dans lequel le dispositif médical (DM) comporte au moins un capteur d'activité du patient (CA) et un module d'interprétation (MI) de ladite activité pour déterminer une donnée d'activité (DA) du patient (P), lesdites données courantes (DC) comportant cette dite donnée d'activité (DA) du patient (P).

6. Programme d'ordinateur (PG) comportant des instructions adaptées pour l'exécution des étapes du procédé de surveillance selon l'une quelconque des revendications 1 à 5 lorsque ledit programme d'ordinateur (PG) est exécuté par au moins un processeur.

7. Support d'enregistrement (CI) lisible par un ordinateur sur lequel est enregistré un programme d'ordinateur (PG) comprenant des instructions pour l'exécution des étapes du procédé de surveillance selon l'une quelconque des revendications 1 à 5.

8. Système de surveillance (100) d'un dispositif médical (DM) à disposition d'un patient (P), ledit dispositif médical (DM) comportant un terminal de communication (T) mettant en œuvre des fonctionnalités logicielles (f1, f2, f3) pour accompagner le patient (P) dans un traitement thérapeutique prédéterminé,
ledit système de surveillance (100) comportant :
- un récepteur (10) configuré pour recevoir du dispositif médical (DM) des données courantes (DC) d'exécution desdites fonctionnalités logicielles (f1, f2, f3),
- un circuit de test (20) configuré pour comparer lesdites données courantes (DC) à des données de référence (DR) selon au moins une règle de vigilance (R) prédéterminée associée à un risque pour le patient (P), ledit risque étant lié à l'exécution d'au moins l'une des fonctionnalités logicielles (f1, f2, f3) avec lesdites données courantes (DC), la règle de vigilance (R) comportant un critère de compatibilité des fonctionnalités logicielles (f1, f2, f3) avec un environnement logiciel et/ou matériel, et
- un émetteur (30) configuré pour émettre :
- un message de vigilance (M1) lorsqu'un tel risque est identifié par le circuit de test (20) lors de l'étape de test (S2), le message de vigilance comportant un ordre pour désactiver à distance l'une au moins desdites fonctionnalités logicielles (f1, f2, f3), et
- un message de validation (M2) lorsqu'aucun risque n'est identifié par le circuit de test (20) lors de l'étape de test (S2), le message de validation (M2) comportant un ordre pour maintenir actives les fonctionnalités logicielles (f1, f2, f3) pendant une période de validité prédéterminée, dans lequel au moins une fonctionnalité logicielle (f1, f2, f3) est désactivée à la fin de la période de validité prédéterminée si aucun autre message de validation (M2) n'a été reçu par le terminal de communication (T).

9. Serveur informatique (200) pour la surveillance d'un dispositif médical (DM) à disposition d'un patient (P), ledit serveur informatique (200) comportant un système de surveillance (100) selon la revendication 8.

10. Dispositif médical (DM) à disposition d'un patient (P) comportant un terminal de communication (T) mettant en œuvre des fonctionnalités logicielles (f1, f2, f3) pour accompagner le patient (P) dans un traitement thérapeutique prédéterminé,
ledit dispositif médical (DM) comportant en outre :
- un moyen de transmission (TRA) configuré pour transmettre des données courantes (DC) d'exécution desdites fonctionnalités logicielles (f1, f2, f3) à destination d'un système de surveillance (100) selon la revendication 8,
- un moyen de vigilance (ACT) configuré pour :
- désactiver au moins une fonctionnalité logicielle (f1, f2, f3) à la réception d'un message de vigilance (M1) émis lorsqu'un risque a été identifié par le système de surveillance (100), ledit message de vigilance (M1) comportant un ordre pour désactiver ladite au moins une fonctionnalité logicielle (f1, f2, f3), et
- maintenir actives les fonctionnalités logicielles (f1, f2, f3) pendant une période de validité prédéterminée à la réception du message de validation (M2) lorsqu'aucun risque n'a été identifié et pour désactiver au moins une fonctionnalité logicielle (f1, f2, f3) lorsqu'aucun autre message de validation (M2) n'a été reçu pendant la période de validité prédéterminée.

11. Dispositif médical (DM) selon la revendication 10, dans lequel le moyen de transmission (TRA) est configuré pour transmettre les données courantes (DC) de façon périodique.

## Patentansprüche

1. Verfahren zur Überwachung eines medizinischen Geräts (DM), das einem Patienten (P) zur Verfügung steht, wobei das medizinische Gerät (DM) ein Kommunikationsendgerät (T) umfasst, das Software-Funktionen (f1, f2, f3) umsetzt, um den Patienten (P) bei einer vorgegebenen therapeutischen Behandlung zu begleiten,
wobei das Verfahren, welches Computer-implementiert ist, die folgenden Schritte umfasst:
- einen Schritt des Empfangens (S1) aktueller Daten (DC) zum Ausführen der Software-Funktionen (f1, f2, f3), wobei die aktuellen Daten (DC) von dem medizinischen Gerät (DM) stammen,
- einen Prüfschritt (S2) der aktuellen Daten (DC) durch Vergleich mit Referenzdaten (DR) gemäß mindestens einer vorbestimmten Wachsamkeitsregel (R), die einem Risiko für den Patienten zugeordnet ist, wobei das Risiko mit der Ausführung mindestens einer der Software-Funktionen (f1, f2, f3) mit den aktuellen Daten (DC) verknüpft ist, wobei die Wachsamkeitsregel (R) ein Kriterium zur Kompatibilität der Software-Funktionen (f1, f2, f3) mit einer Software- oder/und Hardware-Umgebung umfasst, und
- einen Schritt des Sendens (S3) einer Wachsamkeitsnachricht (M1), wenn ein solches Risiko während des Prüfschritts (S2) identifiziert wird, oder einer Bestätigungsnachricht (M2), wenn kein Risiko, identifiziert wird,
wobei die Wachsamkeitsnachricht einen Befehl umfasst, um mindestens eine der Software-Funktionen (f1, f2, f3) aus der Ferne zu deaktivieren, und wobei die Bestätigungsnachricht einen Befehl umfasst, um die Software-Funktionen (f1, f2, f3) für eine vorgegebene Gültigkeitsdauer aktiv zu halten, wobei mindestens eine Software-Funktion (f1, f2, f3) am Ende der vorbestimmten Gültigkeitsdauer deaktiviert wird, sofern keine andere Bestätigungsnachricht (M2) von dem medizinischen Gerät (DM) empfangen wurde.

2. Verfahren nach Anspruch 1, wobei die Wachsamkeitsnachricht (M1) an das medizinische Gerät (DM) gesendet wird.

3. Verfahren nach einem der vorhergehenden Ansprüche, wobei die aktuellen Daten (DC) umfassen:
- eine Information über eine Version der Software-Funktionen (f1, f2, f3), oder/und
- eine Information über die Version der Rechner-Umgebung zur Ausführung der Software-Funktionen (f1, f2, f3), oder/und
- eine Information über das Modell des Kommunikationsendgeräts (T).

4. Verfahren nach einem der vorhergehenden Ansprüche, wobei das medizinische Gerät (DM) neben dem Kommunikationsendgerät (T) ein Zusatzgerät (S) zur medizinischen Messung umfasst und die aktuellen Daten (DC) eine Information zum Modell des Zusatzgeräts (S) umfassen.

5. Verfahren nach einem der vorhergehenden Ansprüche, wobei das medizinische Gerät (DM) mindestens einen Sensor (CA) für eine Aktivität des Patienten und ein Modul (MI) zur Interpretation der Aktivität umfasst, um Daten (DA) zur Aktivität des Patienten (P) zu bestimmen, wobei die aktuellen Daten (DC) die Daten (DA) zur Aktivität des Patienten (P) umfassen.

6. Computerprogramm (PG) mit Anweisungen, die dazu angepasst, sind, die Schritte des Verfahrens zur Überwachung nach einem der Ansprüche 1 bis 5 durchzuführen, wenn das Computerprogramm (PG) von mindestens einem Prozessor ausgeführt wird.

7. Computerlesbarer Datenträger (Cl), auf dem ein Computerprogramm (PG) aufgezeichnet ist, das Anweisungen zur Ausführung der Schritte des Verfahrens zur Überwachung nach einem der Ansprüche 1 bis 5 umfasst.

8. Überwachungssystem (100) für ein medizinisches Gerät (DM), das einem Patienten (P) zur Verfügung steht, wobei das medizinische Gerät (DM) ein Kommunikationsendgerät (T) umfasst, das Software-Funktionen (f1, f2, f3) umsetzt, um den Patienten (P) bei einer vorbestimmten therapeutischen Behandlung zu begleiten,
wobei das Überwachungssystem (100) umfasst:
- einen Empfänger (10), der dazu konfiguriert ist, von dem medizinischen Gerät (DM) aktuelle Daten (DC) zur Ausführung der Software-Funktionen (f1, f2, f3) zu empfangen,
- eine Prüfschaltung (20), die dazu konfiguriert ist, die aktuellen Daten (DC) mit Referenzdaten (DR) gemäß mindestens einer vorbestimmten Wachsamkeitsregel (R) zu vergleichen, die einem Risiko für den Patienten (P) zugeordnet ist, wobei das Risiko mit der Ausführung mindestens einer der Software-Funktionen (f1, f2, f3) mit den aktuellen Daten (DC) verknüpft ist, wobei die Wachsamkeitsregel (R) ein Kriterium zur Kompatibilität der Software-Funktionen (f1, f2, f3) mit einer Software-oder/und Hardware-Umgebung umfasst, und
- einen Sender (30), der dazu konfiguriert ist,
- eine Wachsamkeitsnachricht (M1) zu senden, wenn ein solches Risiko durch die Prüfschaltung (20) im Prüfschritt (S2) identifiziert wird, wobei die Wachsamkeitsnachricht einen Befehl umfasst, um mindestens eine der Software-Funktionen (f1, f2, f3) aus der Ferne zu deaktivieren, und
- eine Bestätigungsnachricht (M2) zu senden, wenn kein Risiko durch die Prüfschaltung (20) im Prüfschritt (S2) identifiziert wird, wobei die Bestätigungsnachricht (M2) einen Befehl umfasst, die Software-Funktionen (f1, f2, f3) für eine vorgegebene Gültigkeitsdauer aktiv zu halten, wobei mindestens eine Software-Funktion (f1, f2, f3) am Ende der vorbestimmten Gültigkeitsdauer deaktiviert wird, sofern keine andere Bestätigungsnachricht (M2) von dem Kommunikationsendgerät (T) empfangen wurde.

9. Computerserver (200) zur Überwachung eines medizinischen Geräts (DM), das einem Patienten (P) zur Verfügung steht, wobei der Computerserver (200) ein Überwachungssystem (100) nach Anspruch 8 umfasst.

10. Medizinisches Gerät (DM), das einem Patienten (P) zur Verfügung steht, umfassend ein Kommunikationsendgerät (T), das Softwarefunktionen (f1, f2, f3) umsetzt, um den Patienten (P) bei einer vorbestimmten therapeutischen Behandlung zu begleiten,
wobei das medizinische Gerät (DM) ferner umfasst:
- ein Übertragungsmittel (TRA), das dazu konfiguriert ist, aktuelle Daten (DC) zum Ausführen der Software-Funktionen (f1, f2, f3) an ein Überwachungssystem (100) nach Anspruch 8 zu übertragen,
- ein Wachsamkeitsmittel (ACT), das dazu konfiguriert ist,
mindestens eine Software-Funktion (f1, f2, f3) beim Empfang einer Wachsamkeitsnachricht (M1) zu deaktivieren, die gesendet wird, wenn ein Risiko durch das Überwachungssystem (100) identifiziert wurde, wobei die Wachsamkeitsnachricht (M1) einen Befehl zum Deaktivieren der mindestens einen Software-Funktion (f1, f2, f3) umfasst, und
bei Empfang einer Bestätigungsnachricht (M2), wenn kein Risiko identifiziert wurde, die Software-Funktionen (f1, f2, f3) für eine vorgegebene Gültigkeitsdauer aktiv zu halten, und dazu, mindestens eine Software-Funktion (f1, f2, f3) zu deaktivieren, sofern keine andere Bestätigungsnachricht (M2) während der vorbestimmten Gültigkeitsdauer empfangen wurde.

11. Medizinisches Gerät (DM) nach Anspruch 10, wobei das Übertragungsmittel (TRA) dazu eingerichtet ist, die aktuellen Daten (DC) periodisch zu übertragen.

## Claims

1. A method for monitoring a medical device (DM) available to a patient (P), wherein said medical device (DM) comprises a communication terminal (T) using software functionalities (f1, f2, f3) for accompanying the patient (P) in a predetermined therapeutic treatment, said method, implemented by computer means, comprising the following steps:
- a step (S1) of receiving current data (DC) for executing said software functionalities (f1, f2, f3), said current data (DC) coming from the medical device (DM),
- a step (S2) of testing said current data (DC) by comparison with reference data (DR) according to at least one predetermined vigilance rule (R) associated with a risk for the patient, said risk being related to the execution of at least one of the software functionalities (f1, f2, f3) with said current data (DC), the vigilance rule (R) comprises a criterion on compatibility of the software functionalities (f1, f2, f3) with a software and/or hardware environment, and
- a step (S3) of sending a vigilance message (M1) when such a risk is identified during the test step (S2) or a validation message (M2) when no risk is identified, wherein the vigilance message includes an instruction for remotely deactivating at least one of said software functionalities (f1, f2, f3) and the validation message includes an instruction for keeping the software functionalities (f1, f2, f3) active for a predetermined period of validity, wherein at least one software functionalities (f1, f2, f3) is deactivated at the end of the predetermined period of validity if no other vigilance message (M2) has been received by the medical device (DM).

2. Method according to claim 1, in which the vigilance message (M1) is sent to the medical device (DM).

3. Method according to any one of the preceding claims, in which the current data (DC) comprises:
- information relating to a version of the software functionalities (f1, f2, f3), et/ou
- information on the computer environment version executing the software functionalities (f1, f2, f3), et/ou
- information on the model of the communication terminal (T).

4. Method according to any one of the preceding claims, in which the medical device (DM) comprises, in addition to the communication terminal (T), an auxiliary medical measurement device (S), and wherein the current data (DC) comprise information on the model of the auxiliary device (S).

5. Method according to any one of the preceding claims, in which the medical device (DM) comprises at least one patient activity sensor (CA) and a module (MI) for interpreting said activity in order to determine an activity data item (DA) on the patient (P), said current data (DC) comprising said activity data item (DA) on the patient (P).

6. Computer program (PG) comprising instructions suitable for executing the steps of the monitoring method according to any one of claims 1 to 5 when said computer program (PG) is executed by at least one processor.

7. Recording medium (CI) that can be read by a computer, on which a computer program (PG) is recorded, comprising instructions for executing the steps of the monitoring method according to any one of claims 1 to 5.

8. System (100) for monitoring a medical device (DM) available to a patient (P), said medical device (DM) comprising a communication terminal (T) using software functionalities (f1, f2, f3) for accompanying the patient (P) in a predetermined therapeutic treatment,
said monitoring system (100) comprising:
- a receiver (10) configured so as to receive from the medical device (DM) current data (DC) for execution of said software functionalities (f1, f2, f3),
- a test circuit (20) configured so as to compare said current data (DC) with reference data (DR) according to at least one predetermined vigilance rule (R) associated with a risk for the patient (P), said risk being related to the execution of at least one of the software functionalities (f1, f2, f3) with said current data (DC), the vigilance rule (R) comprises a criterion on compatibility of the software functionalities (f1, f2, f3) with a software and/or hardware environment, and
- a transmitter (30) configured so as to send :
- a vigilance message (M1) when such a risk is identified by the test circuit (20) during the test step (S2), the vigilance message (M1) including an instruction for remotely deactivating at least one of said software functionalities (f1, f2, f3), and
- a validation message (M2) when no risk is identified by the test circuit (20) during the test step (S2), the validation message (M2) including an instruction for keeping the software functionalities (f1, f2, f3) active for a predetermined period of validity, wherein at least one software functionalities (f1, f2, f3) is deactivated at the end of the predetermined period of validity if no other vigilance message (M2) has been received by the communication terminal (T).

9. Computer server (200) for monitoring a medical device (DM) available to a patient (P), said computer server (200) comprising a monitoring system (100) according to claim 8.

10. Medical device (DM) available to a patient (P), comprising a communication terminal (T) implementing software functionalities (f1, f2, f3) for accompanying the patient (P) in a predetermined therapeutic treatment,
said medical device (DM) further comprising:
- a transmission means (TRA) configured so as to transmit current data (DC) on execution of said software functionalities (f1, f2, f3) to a monitoring system (100) according to claim 8,
- a vigilance means (ACT) configured to:
- deactivate at least one software functionality (f1, f2, f3) on reception of a vigilance message (M1) sent when a risk has been identified by the monitoring system (100), said vigilance message (M1) comprising an instruction for deactivating said at least one software functionality (f1, f2, f3), and
- keeping the software functionalities (f1, f2, f3) active for a predetermined period of validity on reception of a validation message (M2) when no risk has been identified and to deactivate at least one software functionalities (f1, f2, f3) when no other vigilance message (M2) has been received during the predetermined period of validity.

11. Medical device (DM) according to claim 10, in which the transmission means (TRA) is configured to transmit the current data (DC) periodically.
